# EUROPEAN PATENT SPECIFICATION

(11) **EP 1 675 591 B1**
(45) Date of publication and mention of the grant of the patent: **10.08.2011**
(21) Application number: 04790120.2
(22) Date of filing: 05.10.2004
(51) Int. Cl.: A61K 31/46, A61K 31/445, A61P 25/28

(54) **PHARMACEUTICAL COMPOSITION COMPRISING A MONOAMINE NEUROTRANSMITTER RE-UPTAKE INHIBITOR AND AN ACETYLCHOLINESTERASE INHIBITOR**
PHARMAZEUTISCHE ZUSAMMENSETZUNG MIT EINEM MONOAMIN-NEUROTRANSMITTER-WIEDERAUFNAHMEHEMMER UND EINEM ACETYLCHOLINESTERASE-HEMMER
COMPOSITIONS PHARMACEUTIQUES COMPRENANTS DES INHIBITEURS DE RECAPTAGE DE NEUROTRANSMITTEURS MONOAMINE ET DES INHIBITEURS DE L'ACETYLCHOLINESTERASE

(30) Priority: 16.10.2003 EP 03023635; 11.03.2004 EP 04005819
(43) Date of publication of application: 05.07.2006
(60) Divisional application: 10180538.0
(73) Proprietor: NeuroSearch A/S, 2750 Ballerup (DK)
(72) Inventor: FRIEDL, Thomas, 88416 Ochsenhausen (DE); MIERAU, Joachim, 55127 Mainz (DE); RASCHIG, Andreas, 88400 Biberach (DE); REESS, Jürgen, 89075 Ulm (DE); SCHEEL-KRÜGER, Jörgen, DK-2750 Ballerup (DK)
(86) International application number: PCT/EP2004/011093
(87) International publication number: WO 2005/039580

(56) References cited:
- EP-A- 0 604 352
- EP-A- 0 604 354
- EP-A- 0 604 355
- WO-A-00/02549
- WO-A-95/28401
- WO-A-97/30997
- US-A- 5 444 070
- SIMONSON W: "Promising agents for treating Alzheimer's disease" AMERICAN JOURNAL OF HEALTH-SYSTEM PHARMACY, XX, XX, vol. 55, no. SUPPL 2, 1 November 1998 (1998-11-01), pages S11-S16, XP002134583 ISSN: 1079-2082
- DRINGENBERG H C ET AL: "Effect of tacrine on EEG slowing in the rat: enhancement by concurrent monoamine therapy." NEUROBIOLOGY OF AGING. UNITED STATES 2000 JAN-FEB, vol. 21, no. 1, January 2000 (2000-01), pages 135-143, XP001188233 ISSN: 0197-4580
- DRINGENBERG HANS C ET AL: "Electroencephalographic activation by fluoxetine in rats: role of 5-HT(1A) receptors and enhancement of concurrent acetylcholinesterase inhibitor treatment." NEUROPHARMACOLOGY. ENGLAND FEB 2002, vol. 42, no. 2, February 2002 (2002-02), pages 154-161, XP001188231 ISSN: 0028-3908
- GURKA P ET AL: "Pharmacological treatment strategies of residential primary care providers in dementia diseases--results of a representative survey in western Austria." PHARMACOPSYCHIATRY. GERMANY JUL 2002, vol. 35, no. 4, July 2002 (2002-07), pages 144-149, XP008027537 ISSN: 0176-3679
- TODA NARIHIRO ET AL: "A conformational restriction approach to the development of dual inhibitors of acetylcholinesterase and serotonin transporter as potential agents for Alzheimer's disease." BIOORGANIC & MEDICINAL CHEMISTRY, 11 (20) 4389-415., 1 October 2003 (2003-10-01), XP001188230

## Description

### BACKGROUND OF THE INVENTION

### 1. TECHNICAL FIELD

The present invention relates to a combination of a monoamine neurotransmitter re-uptake inhibitor and an acetylcholinesterase inhibitor, and the use of the combination in treating neurodegenerative conditions such as Alzheimer's Disease.

### 2. BACKGROUND INFORMATION

Alzheimer's Disease is an insufficiently understood neurodegenerative condition mainly affecting the elderly but also younger people who are mainly genetically pre-dispositioned to it.

One postulated method of treatment comprises the administration of acetylcholinesterase inhibitors which act on the cholinergic system.

However, this method suffers from the disadvantages that these compounds induce a range of side-effects, especially gastro intestinal discomfort including nausea , diarrhoea and salivation.

Tropane derivative having dopamine reuptake inhibitor activity may in particular be tropane derivatives such as those disclosed by patent applications EP 604355, EP 604352, US 5444070, EP 604354, WO 95/28401, and WO 97/30997.

However, there is no hint to combine these compounds with an acetylcholinesterase inhibitor.

The present invention provides a new and surprisingly effective combination of an acetylcholinesterase inhibitor and for separate, sequential or simultaneous administration of any monoamine neurotransmitter re-uptake inhibitors.

### Surprisingly the combination provides

i) lower doses to be used as expected for the single drugs, and
ii) a reduction or minimization of the adverse event profile of each single drug which increases general tolerability and compliance of both substances and decrease any adverse side effects as the profile of each substance is totally different due to the different mechanism of action.

### BRIEF SUMMARY OF THE INVENTION

Accordingly, the invention relates to a pharmaceutical composition comprising a monoamine neurotransmitter re-uptake inhibitor comprising a 2,3-disubstituted tropane moiety according to formula Ia or a tautomer, a pharmaceutically acceptable salt or a solvate (**1**), and one acetylcholinesterase inhibitor which is donepezil or a pharmaceutically acceptable salt, or a solvate thereof (**2**), and a pharmaceutically acceptable carrier or excipient, and optionally one or more other therapeutic ingredients.

The present invention provides a greater than expected improvement in the condition of subjects suffering from a neurodegenerative disorder with an associated cognitive deficit, such as Alzheimer's Disease, Lewis body disease, fronto-temporal dementia, or from a cognitive deficit which may arise from a normal process such as aging like cerebrovascular dementia and milder forms as age associated memory impairment (AAMI) or mild cognitive impairment (MCI) or from an abnormal process such as injury, than would be expected from administration of the active ingredients alone. Further, the combination allows for a lower overall dose of each of the active ingredients to be administered thus reducing side effects and decreasing any reduction in the effectiveness of each of the active ingredients over time.

There is also provided a kit of parts comprising at least two separate unit dosage forms (A) and (B):
(A) one of which comprises a composition a monoamine neurotransmitter re-uptake inhibitor comprising a 2,3-disubstituted tropane moiety P2 ① or a tautomer, a pharmaceutically acceptable salt, or a solvate thereof (1), and optionally a pharmaceutically acceptable carrier;
(B) one of which comprises a composition containing one acetylcholinesterase inhibitor P2 ② or a pharmaceutically acceptable salt, or a solvate thereof **(2),** and optionally a pharmaceutically acceptable carrier,
   for simultaneous, sequential or separate administration.

There is also provided the use of a combination of a monoamine neurotransmitter re-uptake inhibitor comprising a 2,3-disubstituted tropane moiety P2 ①, or a tautomer, a pharmaceutically acceptable salt, solvate thereof (1) and one acetylcho linesterase inhibitor P2 ② or a pharmaceutically acceptable salt, or solvate thereof (2) in a combined form, or separately or separately and sequentially, wherein the sequential administration is close in time or remote in time, for the manufacture of a medicamentation for the prevention or treatment of a disease or a disorder, which is responsive to the inhibition of monoamine neurotransmitter re-uptake and or to AChE inhibition.

### DETAILED DESCRIPTION OF THE INVENTION

The term "pharmaceutically acceptable acid addition salt" as used herein includes those salts which are selected from among the acid addition salts formed with hydrochloric acid, hydrobromic acid, sulphuric acid, phosphoric acid, methanesulphonic acid, acetic acid, fumaric acid, succinic acid, lactic acid, citric acid, tartaric acid and maleic acid, the salts obtained from hydrochloric acid, hydrobromic acid, sulphuric acid, phosphoric acid and acetic acid being particularly preferred. The salts of citric acid are of particular significance.

The monoamine neurotransmitter re-uptake inhibitor of the invention is a compound of formula (IA) 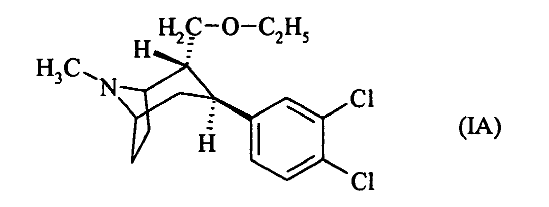 or a pharmaceutically acceptable salt thereof, in particular the citrate thereof.

The Acetylcholinesterase inhibitor of the invention is donepezil and its hydrochloride.

The pharmaceutical compositions of the present invention are suitable for oral, intravenous, intravascular, intraperitoneal, subcutaneous, intramuscular, inhalativ, topical, patch or suppository administration.

The pharmaceutical compositions of the present invention are preferably in unit dosage forms such as tablets, pills, capsules, powders, granules, sterile parenteral solutions or suspensions, metered aerosol or liquid sprays, drops, ampoules, transdermal patches, auto-injector devices or suppositories; for oral, parenteral, intranasal, sublingual or rectal administration, or for administration by inhalation or insufflation. For preparing solid compositions such as tablets, the principal active ingredient is mixed with a pharmaceutical carrier, e. g. conventional tableting ingredients such as corn starch, cellulose, carboxymethylcellulose, hydroxypropylmethylcellulose, lactose, sucrose, sorbitol, talc, silicon dioxide, polyethylene glycol, stearic acid, magnesium stearate and dicalcium phosphate or gums or surfactants such as sorbitan monooleate, polyethylene glycol, and other pharmaceutical diluents, e. g. water, to form a solid pre-formulation composition containing a homogeneous mixture of a compound of the present invention, or a pharmaceutically acceptable salt thereof. When referring to these pre-formulation compositions as homogeneous, it is meant that the active ingredient is dispersed evenly throughout the composition so that the composition may be readily subdivided into equally effective unit dosage forms such as tablets, pills and capsules.

This solid pre-formulation composition is then subdivided into unit dosage forms of the type described above containing from 0.05 to 10,000 mg, in particular 0.1 to about 500 mg, most preferably 0.1 to 250 mg of each active ingredient of the present invention. Typical unit dosage forms contain from 0.1 to 100 mg, for example 0.1, 0.5, 1, 2, 5, 10, 25, 50 or 100 mg, of each active ingredient. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

Similarly, cachets and lozenges are included. Tablets, powders, capsules, pills, cachets, and lozenges can be used as solid forms suitable for oral administration.

The liquid forms in which the novel compositions of the present invention may be incorporated for administration orally or by injection include aqueous solutions, suitably flavored syrups, aqueous or oil suspensions, and flavored emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinyl-pyrrolidone or gelatin.

For preparing suppositories, a low melting was, such as admixture of fatty acid glycerides or cocoa butter, is first melted and the active component is dispersed homogeneously therein, as by stirring. The molten homogenous mixture is then poured into convenient sized molds, allowed to cool, and thereby to solidify.

Formulations suitable for vaginal administration may be presented as pessaries, tampons, creams, gels, pastes, foams or sprays containing in addition to the active ingredient such carriers as are known in the art to be appropriate.

Administration to the respiratory tract may also be achieved by means of an aerosol formulation in which the active ingredient is provided in a pressurised pack with a suitable propellant such as a chlorofluorocarbon (CFC) or fluorohydrocarbon (HFC) for example dichlorodifluoromethane, trichlorofluoromethane, dichlorotetrafluoroethane, 1,1,1,2-tetrafluoroethan (HFC-134(a)), or 1,1,1,2,3,3,3-heptafluoroprpane, carbon dioxide, or other suitable gas. The aerosol may conveniently also contain a surfactant such as lecithin and/or a co-solvent such as ethanol. The dose of drug may be controlled by provision of a metered valve.

Alternatively the active ingredients may be provided in the form of a dry powder, for example a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, e.g., gelatin, or blister packs from which the powder may be administered by means of an inhaler.

In formulations intended for administration to the respiratory tract, including intranasal formulations, the compound will generally have a small particle size for example of the order of 5 microns or less. Such a particle size may be obtained by means known in the art, for example by micronization.

Preferably the weight ratio of (**1**) to (**2**) ranges from 50 : 1 to 1 : 300, in particular from 1 : 1 to 1 : 200 most preferably from 1 : 2 to 1 : 100.

Most preferred are the following daily dose rates:
- 0.5 - 20 mg, preferably 1.0 - 10 mg of donepezil and 0.01 - 2.0 mg of the compound of formula (IA);

The Example that follow serves to illustrate a formulation according to the invention. It is intended solely as a possible procedure described by way of example

### Example 1 Composition of (IA) / Donepezil film-coated tablet 0.5 mg / 5 mg

### Core

| **Constituents** | **mg/tablet** |
|---|---|
| (IA) citrate | 0.793 |
| Donepezil hydrochloride | 5.482 |
| Lactose monohydrate (200 mesh) | 98.125 |
| Microcrystalline cellulose (grade PH 101) | 63.000 |
| Corn starch | 6.300 |
| Purified water | (q.s.)* |
| Sodiumstarchglycolate | 3.600 |
| Colloidal silicon dioxide | 0.900 |
| Magnesium stearate | 1.800 |

### Coating

| **Constituents** | **mg/ tablet** |
|---|---|
| Hydroxyproylmethylcellulose 2910 | 2.750 |
| Polyethylene Glycol 400 | 0.325 |
| Titanium dioxide | 1.000 |
| Talc | 0.925 |
| Purified water | (q.s.)* |
| **Total weight film coated tablet** | **185.000** |

| | |
|---|---|
| ** does not appear in final product* | |

The advantageous effect of the combination of the present invention can be shown, for example, by comparing the combined dosage of the combination with dosages of the same amount of each of the active ingredients separately on subjects using the Mini-Mental State Examination (MMSE) as described in Folstein and Folstein J. Psychiat. Res., 1975,12,189-198 or a variant thereof as discussed in Tornbaugh and McIntyre, JAGS, 1992,40,922-935.

## Claims

1. A pharmaceutical composition comprising a monoamine neurotransmitter re-uptake inhibitor comprising a 2,3-disubstituted tropane moiety being a compound of formula (IA) 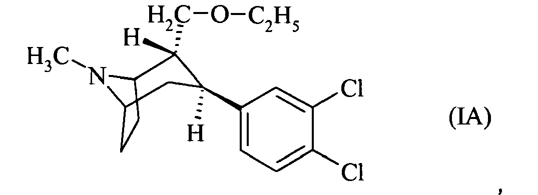 or a tautomer, a pharmaceutically acceptable salt, or solvate thereof **(1),** and donepezil or a pharmaceutically acceptable salt, or solvate thereof **(2),** and a pharmaceutically acceptable carrier or excipient, and optionally one or more other therapeutic ingredients.

2. A pharmaceutical composition according to claim 1 consisting essentially of the compound of formula (IA) 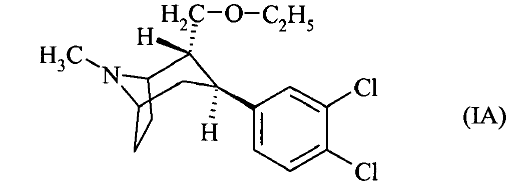 or a pharmaceutically acceptable salt thereof, (1) and donepezil or a pharmaceutically acceptable salt, or solvate thereof (2), and a pharmaceutically acceptable carrier or excipient.

3. A pharmaceutical formulation according to claim 1 or 2 which is suitable for oral, intra venous, intravascular, intraperitoneal, subcutaneous, intramuscular or topical or patch or suppository administration.

4. A pharmaceutical formulation according to any of claims 1 to 3 wherein the weight ratio of **(1)** to **(2)** ranges from 50:1 to 1:300.

5. A pharmaceutical formulation according to any of claims 1 to 4 wherein a single application dose contains 0.05 to 10,000 milligrams of the combined active ingredients **(1)** and **(2).**

6. A pharmaceutical formulation according to any of claims 1 to 5 wherein the pharmaceutically acceptable carrier or excipient is selected from the group consisting of corn starch, cellulose, carboxymethylcellulose, hydroxypropylmethylcellulose, lactose, sucrose, sorbitol, talc, silicon dioxide, polyethylene glycol, stearic acid, magnesium stearate and dicalcium phosphate.

7. Use of a monoamine neurotransmitter re-uptake inhibitor comprising a 2,3-disubstituted tropane moiety being a compound of formula (IA) 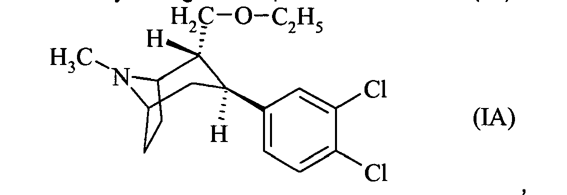 or a tautomer, a pharmaceutically acceptable salt, or solvate thereof **(1)** and donepezil or a pharmaceutically acceptable salt, or solvate thereof **(2)** in a combined form, or separately or separately and sequentially, wherein the sequential administration is close in time or remote in time, for the manufacture of a medicamentation for the prevention or treatment of a disease or a disorder, which is selected from the group consisting of pseudodementia, dementia, including dementia of Alzheimer Type, Alzheimer's disease, presenile dementia, senile dementia, Lewy-Body-dementia, Down syndrome, fronto temporal dementia, HIV related dementia, Pick's disease, multi-infarct dementia, memory deficits, attention deficits, cognitive dysfunction, memory dysfunction, mild cognitive impairment, age associated memory impairment, ageing-associated cognitive decline, age-related cognitive decline and multiple system atrophy.

8. Use according to claim 7 wherein the disease or disorder is dementia of Alzheimer Type.

9. A pharmaceutical kit comprising at least two separate unit dosage forms (A) and (B):
(A) one of which comprises a composition comprising a monoamine neurotransmitter re-uptake inhibitor comprising a 2,3-disubstituted tropane moiety being a compound of formula (IA) 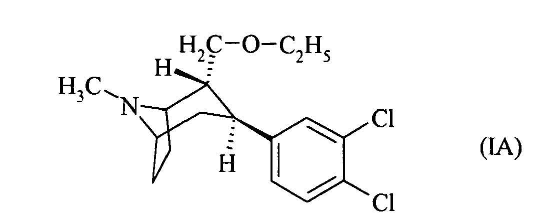 or a tautomer, a pharmaceutically acceptable salt, or solvate thereof **(1),** and optionally a pharmaceutically acceptable carrier;
(B) one of which comprises a composition containing donepezil or a pharmaceutically acceptable salt, or solvate thereof **(2),** and optionally a pharmaceutically acceptable carrier.

## Patentansprüche

1. Pharmazeutische Zusammensetzung, umfassend einen Monoamin-Neurotransmitter-Wiederaufnahmehemmer, umfassend eine 2,3-disubstituierte Tropaneinheit, bei der es sich um eine Verbindung der Formel (IA) handelt 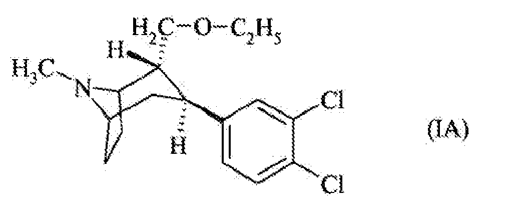 oder ein Tautomer, ein pharmazeutisch verträgliches Salz oder Solvat davon (1), und Donepezil oder ein pharmazeutisch verträgliches Salz oder Solvat davon (**2**), und einen pharmazeutisch verträglichen Träger oder Exzipienten, und gegebenenfalls einen oder mehrere andere therapeutische Inhaltsstoffe.

2. Pharmazeutische Zusammensetzung nach Anspruch 1, bestehend im Wesentlichen aus der Verbindung der Formel (IA) 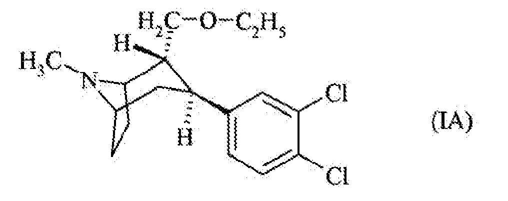 oder einem pharmazeutisch verträglichen Salz davon (**1**) und Donepezil oder einem pharmazeutisch verträglichen Salz oder Solvat davon (**2**), und einem pharmazeutisch verträglichen Träger oder Exzipienten.

3. Pharmazeutische Formulierung nach Anspruch 1 oder 2, welche für eine orale, intravenöse, intravaskuläre, intraperitoneale, subkutane, intramuskuläre oder topische Verabreichung oder Verabreichung als Pflaster oder Zäpfchen geeignet ist.

4. Pharmazeutische Formulierung nach einem der Ansprüche 1 bis 3, wobei das Gewichtsverhältnis von (**1**) zu (**2**) im Bereich von 50:1 bis 1:300 liegt.

5. Pharmazeutische Formulierung nach einem der Ansprüche 1 bis 4, wobei eine Einzelanwendungsdosis 0,05 bis 10000 Milligramm der kombinierten Wirkstoffe (**1**) und (**2**) enthält.

6. Pharmazeutische Formulierung nach einem der Ansprüche 1 bis 5, wobei der pharmazeutisch verträgliche Träger oder Exzipient ausgewählt ist aus der Gruppe bestehend aus Maisstärke, Cellulose, Carboxymethylcellulose, Hydroxypropylmethylcellulose, Lactose, Sucrose, Sorbitol, Talk, Siliciumdioxid, Polyethylenglycol, Stearinsäure, Magnesiumstearat und Dicalciumphosphat.

7. Verwendung eines Monoamin-Neurotransmitter-Wiederaufnahmehemmers, umfassend eine 2,3-disubstituierte Tropaneinheit, bei der es sich um eine Verbindung der Formel (IA) handelt 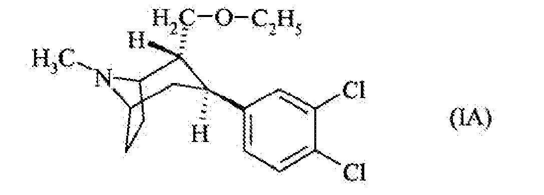 oder ein Tautomer, ein pharmazeutisch verträgliches Salz oder Solvat davon (**1**) und Donepezil oder ein pharmazeutisch verträgliches Salz oder Solvat davon (**2**) in einer kombinierten Form, oder getrennt oder getrennt und sequenziell, wobei die sequenzielle Verabreichung zeitlich nah oder zeitlich entfernt ist, für die Herstellung eines Medikaments für die Verhütung oder Behandlung einer Krankheit oder einer Störung, welche ausgewählt ist aus der Gruppe bestehend aus Pseudodemenz, Demenz, einschließlich Demenz vom Alzheimer-Typ, Alzheimer-Krankheit, präseniler Demenz, seniler Demenz, Lewy-Körper-Demenz, Down-Syndrom, frontotemporaler Demenz, Demenz in Verbindung mit HIV, Pick-Syndrom, Multiinfarktdemenz, Gedächtnisdefiziten, Aufmerksamkeitsdefiziten, kognitiver Dysfunktion, Gedächtnisdysfunktion, milder kognitiver Beeinträchtigung, mit dem Alter zusammenhängender Beeinträchtigung des Gedächtnisses, mit dem Altern zusammenhängender kognitiver Verschlechterung, altersbedingter kognitiver Verschlechterung und multipler Systematrophie.

8. Verwendung nach Anspruch 7, wobei die Krankheit oder Störung eine Demenz vom Alzheimer-Typ ist.

9. Pharmazeutisches Kit, umfassend wenigstens zwei getrennte Dosiseinheitsformen (A) und (B):
(A) eine von diesen umfasst eine Zusammensetzung, umfassend einen Monoamin-Neurotransmitter-Wiederaufnahmehemmer, umfassend eine 2,3-disubstituierte Tropaneinheit, bei der es sich um eine Verbindung der Formel (IA) handelt 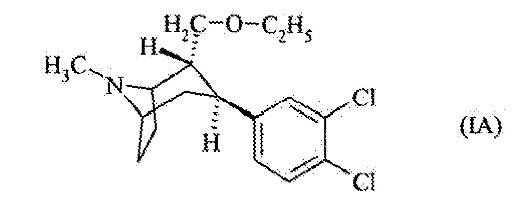 oder ein Tautomer, ein pharmazeutisch verträgliches Salz oder Solvat davon (**1**), und gegebenenfalls einen pharmazeutisch verträglichen Träger;
(B) eine von diesen umfasst eine Zusammensetzung, enthaltend Donepezil oder ein pharmazeutisch verträgliches Salz oder Solvat davon (**2**), und gegebenenfalls einen pharmazeutisch verträglichen Träger.

## Revendications

1. Composition pharmaceutique comprenant un inhibiteur de la recapture des neurotransmetteurs monoamines comprenant un fragment tropane 2,3-disubstitué qui est un composé de formule (IA) 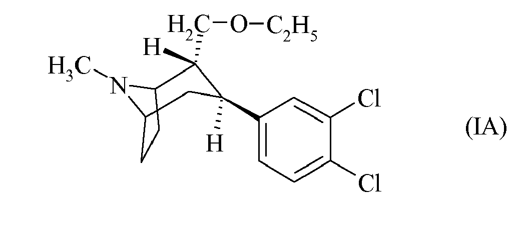 ou un tautomère, un sel pharmaceutiquement acceptable ou un solvate de celui-ci **(1),** et du donépézil ou un sel pharmaceutiquement acceptable ou un solvate de celui-ci **(2),** et un support ou excipient pharmaceutiquement acceptable, et facultativement un ou plusieurs autres ingrédients thérapeutiques.

2. Composition pharmaceutique selon la revendication 1, constituée essentiellement du composé de formule (IA) 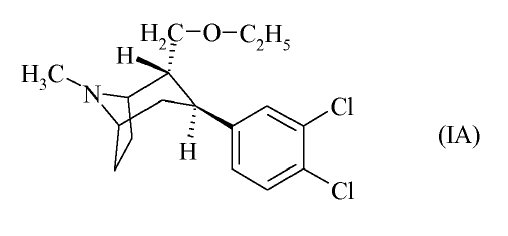 ou d'un sel pharmaceutiquement acceptable de celui-ci **(1),** et de donépézil ou d'un sel pharmaceutiquement acceptable ou solvate de celui-ci **(2),** et d'un support ou excipient pharmaceutiquement acceptable.

3. Formulation pharmaceutique selon la revendication 1 ou 2, qui est appropriée pour une administration orale, intraveineuse, intravasculaire, intrapéritonéale, sous-cutanée, intramusculaire ou topique ou par timbre ou par suppositoire.

4. Formulation pharmaceutique selon l'une quelconque des revendications 1 à 3, dans laquelle le rapport en poids entre **(1)** et **(2)** varie de 50:1 à 1:300.

5. Formulation pharmaceutique selon l'une quelconque des revendications 1 à 4, dans laquelle une seule dose d'application contient de 0,05 à 10 000 milligrammes des ingrédients actifs **(1)** et **(2)** combinés.

6. Formulation pharmaceutique selon l'une quelconque des revendications 1 à 5, dans laquelle le support ou excipient pharmaceutiquement acceptable est choisi dans le groupe constitué par l'amidon de maïs, la cellulose, la carboxyméthylcellulose, l'hydroxypropylméthylcellulose, le lactose, le saccharose, le sorbitol, le talc, le dioxyde de silicium, le poly(éthylène glycol), l'acide stéarique, le stéarate de magnésium et le phosphate dicalcique.

7. Utilisation d'un inhibiteur de la recapture des neurotransmetteurs monoamines comprenant un fragment tropane 2,3-disubstitué qui est un composé de formule (IA) 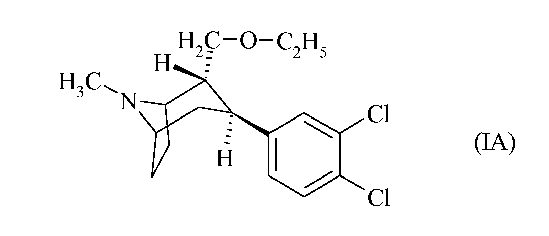 ou un tautomère, un sel pharmaceutiquement acceptable ou un solvate de celui-ci **(1),** et du donépézil ou un sel pharmaceutiquement acceptable ou un solvate de celui-ci **(2),** sous une forme combinée, ou séparément ou séparément et séquentiellement, où l'administration séquentielle est proche dans le temps ou éloignée dans le temps, pour la fabrication d'une médication destinée à la prévention ou au traitement d'une maladie ou d'un trouble, qui est choisi dans le groupe constitué par la pseudodémence, la démence, y compris la démence de type Alzheimer, la maladie d'Alzheimer, la démence présénile, la démence sénile, la démence à corps de Lewy, le syndrome de Down, la démence frontotemporale, la démence liée au VIH, la maladie de Pick, la démence artériopathique, les déficits de la mémoire, les déficits de l'attention, un dysfonctionnement cognitif, un dysfonctionnement de la mémoire, un trouble cognitif modéré, un trouble de la mémoire associé à l'âge, un déclin cognitif associé au vieillissement, un déclin cognitif lié à l'âge et une atrophie multisystématisée.

8. Utilisation selon la revendication 7, dans laquelle la maladie ou le trouble est une démence de type Alzheimer.

9. Nécessaire pharmaceutique comprenant au moins deux formes posologiques unitaires séparées (A) et (B) :
(A) dont l'une comprend une composition comprenant un inhibiteur de la recapture des neurotransmetteurs monoamines comprenant un fragment tropane 2,3-disubstitué étant un composé de formule (IA) 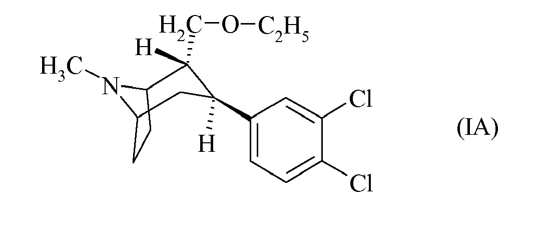 ou un tautomère, un sel pharmaceutiquement acceptable ou un solvate de celui-ci **(1),** et facultativement un support pharmaceutiquement acceptable ;
(B) dont l'autre comprend une composition contenant du donépézil ou un sel pharmaceutiquement acceptable ou solvate de celui-ci **(2),** et facultativement un support pharmaceutiquement acceptable.
